# EUROPÄISCHE PATENTSCHRIFT

(11) **EP 0 980 351 B1**
(45) Veröffentlichungstag und Bekanntmachung des Hinweises auf die Patenterteilung: **18.02.2004**
(21) Anmeldenummer: 98925500.5
(22) Anmeldetag: 29.04.1998
(51) Int. Cl.: C07C 217/58, C07C 217/60, C07C 317/22, C07C 323/20, C07C 271/16, C07D 295/18, A61K 31/135, A61K 31/445, C07H 15/203

(54) **BENZYLAMINDERIVATE UND PHENYLETHYLAMINDERIVATE, VERFAHREN ZU IHRER HERSTELLUNG UND IHRE VERWENDUNG ALS ARZNEIMITTEL**
BENZYLAMINE AND PHENYLETHYLAMINE DERIVATIVES, PROCESSES FOR PREPARING THE SAME AND THEIR USE AS MEDICAMENTS
DERIVES DE BENZYLAMINE ET DE PHENYLETHYLAMINE, LEURS PROCEDES DE PREPARATION ET LEUR UTILISATION COMME MEDICAMENTS

(30) Priorität: 30.04.1997 DE 19718334
(43) Veröffentlichungstag der Anmeldung: 23.02.2000
(73) Patentinhaber: Boehringer Ingelheim Pharma GmbH & Co.KG, 55218 Ingelheim am Rhein (DE)
(72) Erfinder: ANDERSKEWITZ, Ralf, D-55411 Bingen (DE); SCHROMM, Kurt, D-55218 Ingelheim am Rhein (DE); RENTH, Ernst-Otto, D-24105 Kiel (DE); BIRKE, Franz, D-55218 Ingelheim am Rhein (DE); JENNEWEIN, Hans, Michael, D-65193 Wiesbaden (DE); MEADE, Christopher, John, Montague, D-55411 Bingen (DE)
(74) Vertreter: Laudien, Dieter, Dr.
(86) Internationale Anmeldenummer: PCT/EP1998/002530
(87) Internationale Veröffentlichungsnummer: WO 1998/049131

(56) Entgegenhaltungen:
- DE-A- 4 424 713
- DE-A- 4 424 714

## Beschreibung

Die vorliegende Erfindung betrifft neue Benzylaminderivate sowie Phenylethylaminderivate, Verfahren zu ihrer Herstellung sowie ihre Verwendung als Arzneimittel.

Die deutsche Patentanmeldung DE-A-4424713 schlägt Verbindungen mit LTB₄-rezeptorantagonistischer Wirkung vor, die anstelle einer -CHR₃R₄ Gruppe eine Amidin Gruppe aufweisen. Für den Fachmann gab es keine Veranlassung, eine Benzamidingruppe durch eine -CHR₃R₄ Gruppe zu ersetzen.

Die erfindungsgemäßen Benzyl- bzw. Phenylethylaminderivate entsprechen der allgemeinen Formel I 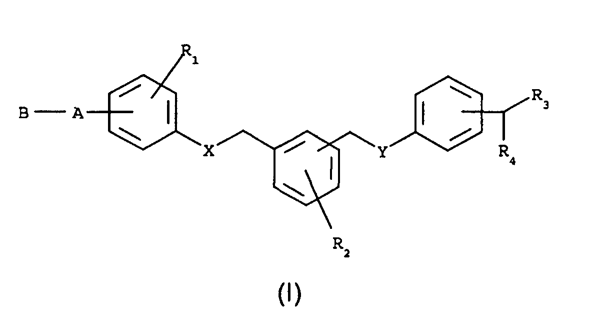 worin
- X: O, NH, N(CH₃), CH₂;
- Y: O, NH, N(CH₃), CH₂;
- R₁: H, F, Cl, Br, J, C₁-C₆-Alkyl, OH, O-C₁-C₆-Alkyl, CF₃;
- R₂: H, F, Cl, Br, J, C₁-C₆-Alkyl, OH, O-C₁-C₆-Alkyl, CF₃;
- R₃: H, NH₂, NHCOR₅;
- R₄: H, CH₂NH₂, CH₂NHCOR₅;
- R₅: H, C₁-C₆-Alkyl, Phenyl, O-(C₁-C₆-Alkyl), wobei der Phenylring bis zu zweimal substituiert sein kann durch: F, Cl, Br, J, Rₐ, ORₐ, CF₃,
- Rₐ: H, C₁-C₆-Alkyl;
- A: CR₆R₇, CO, SOₓ, O;
- x: eine ganze Zahl 0, 1 oder 2;
- R₆: H, C₁-C₄-Alkyl, C₃-C₆-Cycloalkyl, -(CH₂)_{y}COOR₈, CF₃, -(CH₂)_{y}OR₈, OR₈;
- y: eine ganze Zahl 0, 1 , 2, 3 oder 4;
- R₇: H, C₁-C₄-Alkyl, C₃-C₆-Cycloalkyl, -(CH₂)_{z}COOR₈, -(CH₂)_{z}OR₈, CF₃;
- z: eine ganze Zahl 0, 1, 2, 3 oder 4,
wobei R₆ und R₇ miteinander ggf. einen C₃-C₆-Cycloalkylring bilden können;
- R₈: H, C₁-C₆-Alkyl;
- B: C₁-C₆-Alkyl, CONR₉R₁₀, Ar, und, wenn A die Bedeutung -C(CH₃)₂ hat: CH₂NR₉R₁₀, CH₂NR₉COR₁₁;
- Ar: Phenyl, Naphthyl, Thienyl, Pyridyl - gegebenfalls bis zu zweimal substituiert mit R₁₂,
- R₉: H, C₁-C₆-Alkyl;
- R₁₀: H, C₁-C₆-Alkyl, wobei R₉ und R₁₀ zusammen mit dem Stickstoffatom einen Ring mit 3 bis 7 Kohlenstoffatomen bilden können;
- R₁₁: H, C₁-C₆-Alkyl, -O-(C₁-C₆-Alkyl), Phenyl;
- R₁₂: H, C₁-C₆-Alkyl, O-(C₁-C₆-Alkyl), F, Cl, Br, J, Rₐ, CF₃, CHF₂, C(CH₃)₂-phenylen-OH, COORₐ, CONRₐR_{b}, OR_{c};
- Rₐ: H, C₁-C₆-Alkyl-,
- R_{b}: H, C₁-C₆-Alkyl, wobei ggf. Rₐ und R_{b} zusammen mit dem Stickstoffatom einen Ring mit 3 bis 7 Kohlenstoffatomen bilden können;
- R_{c}: für H, C₁-C₆-Alkyl, COOR_{d}, COR_{d} oder eine Gruppe der Formel 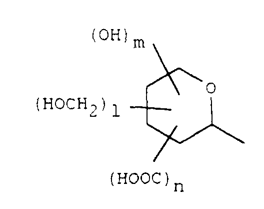 l, m, n eine ganze Zahl 0, 1, 2, 3 oder 4 wobei gilt l+m+n≤ 4;
- R_{d}: C₁-C₆-Alkyl, Phenyl,
bedeuten können - gegebenenfalls in Form der einzelnen optischen Isomeren, Mischungen der einzelnen Enantiomeren oder Racemate sowie in Form der freien Basen oder der enstsprechenden Säureadditionssalze mit pharmakologisch unbedenklichen Säuren - mit der Maßgabe daß

R₃ und R₄ beide zusammen nicht Wasserstoff bedeuten können.

Bevorzugt sind Verbindungen der allgemeinen Formel I in der
- X: O;
- Y: O;

- R₁: H, F, Cl, Br, J, C₁-C₆-Alkyl, OH, O-C₁-C₆-Alkyl, CF₃;
- R₂: H, F, Cl, Br, J, C₁-C₆-Alkyl, OH, O-C₁-C₆-Alkyl, CF₃;
- R₃: NH₂;
- R₄: H;

- A: CR₆R₇, O;
- R₆: H, C₁-C₄-Alkyl, CF₃;
- R₇: H, C₁-C₄-Alkyl, CF₃,
wobei R₆ und R₇ miteinander ggf. einen C₃-C₆-Cycloalkylring bilden können;
- B: Phenyl, ggf. bis zu zweimal substituiert mit F, Cl, Br, J, Rₐ, OR_{c}, CF₃;
- Rₐ: H, C₁ -C₆-Alkyl;
- R_{c}: für H, C₁-C₆-Alkyl, COOR_{d}, COR_{d} oder eine Gruppe der Formel 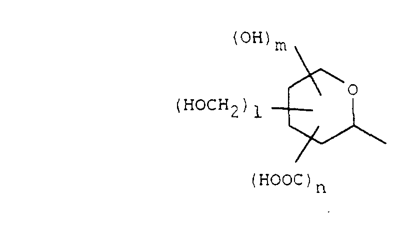 l, m, n eine ganze Zahl 0, 1, 2, 3 oder 4 wobei gilt l+m+n≤4;
- R_{d}: C₁-C₆-Alkyl, Phenyl
bedeuten können - gegebenenfalls in Form der einzelnen optischen Isomeren, Mischungen der einzelnen Enantiomeren oder Racemate sowie in Form der freien Basen oder der entsprechenden Säureadditionssalze mit pharmakologisch unbedenklichen Säuren.

Soweit nicht im einzelnen abweichende Angaben gemacht werden, werden die allgemeinen Definitionen im folgenden Sinn gebraucht:
C₁-C₄-Alkyl, C₁-C₆-Alkyl bzw. C₁-C₈-Alkyl steht im allgemeinen für einen verzweigten oder unverzweigten Kohlenwasserstoffrest mit 1 bis 4 oder 6 bzw. 8 Kohlenstoffatom(en), der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:
   Methyl, Ethyl, Propyl, 1-Methylethyl (Isopropyl), n-Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylproypyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2,-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Bevorzugt sind - sofern nicht anders angegeben - Niederalkylreste mit 1 bis 4 Kohlenstoffatomen, wie Methyl; Ethyl, Propyl, *iso*-Propyl, n-Butyl, 1-Methylpropyl, 2-Methylpropyl oder 1,1-Dimethylethyl.

Wie gefunden wurde, zeichnen sich die erfindungsgemäßen Verbindungen der Formel I durch vielfältige Anwendungsmöglichkeiten auf therapeutischem Gebiet sowie durch ihre orale Wirksamkeit aus. Hervorzuheben sind solche Anwendungsmöglichkeiten, für welche die LTB₄-rezeptorantagonistischen Eigenschaften eine Rolle spielen. Hier sind insbesondere zu nennen:
Arthritis, Asthma, chronische obstruktive Lungenerkrankungen, etwa chronische Bronchitis, Psoriasis, Colitis ulcerosa, durch nichtsteroidale Antiphlogistika induzierte Gastro- oder Enteropathie, cystische Fibrose, Alzheimer-Krankheit, Schock, Reperfusionsschäden/lschämien, Atherosklerose, Multiple Sklerose.

Auch lassen sich mit den neuen Verbindungen Krankheiten oder Zustände behandeln, bei denen die Passage von Zellen aus dem Blut über das vaskuläre Endothelium in das Gewebe von Bedeutung ist (etwa Metastasis) oder Krankheiten und Zustände, bei denen die Kombination des LTB₄ oder eines anderen Moleküls (beispielsweise 12-HETE) mit dem LTB₄-Rezeptor einen Einfluß auf die Zell-Proliferation hat (etwa chronische myelozytische Leukämie).

Die neuen Verbindungen können auch in Kombination mit anderen Wirkstoffen angewendet werden, etwa solchen, die für dieselben Indikationen Verwendung finden, oder z.B. mit Antiallergika, Sekretolytika, β₂-Adrenergika, inhalativ anwendbaren Steroiden, Antihistaminika und/oder PAF-Antagonisten, NSAID sowie Glucocorticoide. Die Verabreichung kann topisch, oral, transdermal, nasal, parenteral oder inhalativ erfolgen.

Zur pharmakologischen und biochemischen Untersuchung der Wirkungsverhältnisse eigenen sich Tests, wie sie beispielsweise in der WO 93/16036, S. 15 bis 17 - auf die hier inhaltlich Bezug genommen wird - dargestellt sind.

Die therapeutische oder prophylaktische Dosis ist - außer von der Wirkungsstärke der einzelnen Verbindungen und dem Körpergewicht des Patienten - abhängig von der Beschaffenheit und Ernsthaftigkeit des Krankheitszustandes. Bei oraler Anwendung liegt die Dosis zwischen 1 und 500 mg, vorzugsweise zwischen 20 und 250 mg. Bei inhalativer Anwendung werden dem Patienten zwischen etwa 0,5 und 25, vorzugsweise zwischen etwa 2 und 20 mg Wirkstoff zugeführt.

Inhalationslösungen enthalten im allgemeinen zwischen etwa 0,5 und 5 % Wirkstoff. Die neuen Verbindungen können in üblichen Zubereitungen verabreicht werden, etwa als Tabletten, Dragées, Kapseln, Oblaten, Pulver, Granulate, Lösungen, Emulsionen, Sirupe, Inhalationsaerosole, Salben, Suppositorien.

Die nachstehenden Beispiele zeigen einige Möglichkeiten für die Formulierung der Darreichungsformen:

### Formulierungsbeispiele

### 1. Tabletten

| Zusammensetzung: | |
|---|---|
| Wirkstoff gemäß der Erfindung | 20 Gew.-Teile |
| Stearinsäure | 6 Gew.-Teile |
| Traubenzucker | 474 Gew.-Teile |

Die Bestandteile werden in üblicher Weise zu Tabletten von 500 mg Gewicht verarbeitet. Gewünschtenfalls kann der Wirkstoffgehalt erhöht oder vermindert und die Traubenzuckermenge entsprechend vermindert oder erhöht werden.

### 2. Suppositorien

| Zusammensetzung: | |
|---|---|
| Wirkstoff gemäß der Erfindung | 100 Gew.-Teile |
| Laktose, gepulvert | 45 Gew.-Teile |
| Kakao-Butter | 1555 Gew.-Teile |

Die Bestandteile werden in üblicher Weise zu Suppositorien von 1,7 g Gewicht verarbeitet.

### 3. Inhalationspulver

Mikronisiertes Wirkstoffpulver (Verbindung der Formel I; Teilchengröße ca. 0,5 bis 7 µm) werden in einer Menge von 5 mg gegebenenfalls unter Zusatz mikronisierter Lactose in Hartgelatinekapseln abgefüllt. Das Pulver wird aus üblichen Inhalationsgeräten, z.B. gemäß DE-A 33 45 722, auf die hiermit inhaltlich Bezug genommen wird, inhaliert.

Die erfindungsgemäßen Verbindungen sind ausgehend von aus dem Stand der Technik bekannten Verbindungen u.a. nach den in den folgenden Beispielen beschriebenen Verfahren herstellbar. Verschiedenartige, andere Ausgestaltungen der Verfahren werden für den Fachmann aus der vorliegenden Beschreibung ersichtlich. Es wird jedoch ausdrücklich darauf hingewiesen, daß diese Beispiele und die diesen zugeordnete Beschreibung lediglich zum Zweck der Erläuterung vorgesehen und nicht als Einschränkung der Erfindung anzusehen sind.

### Synthesebeispiele

Die erfindungsgemäßen Verbindungen können aus den entsprechenden Nitril-Verbindungen durch Reduktion erhalten werden, entweder durch katalytische Hydrierung in Solventien wie Methanol, Ethanol, höheren Alkoholen, DMF, Wasser mit Katalysatoren wie Raney-Nickel, Pd/C, Platin, oder mit Hydrid-Reagenzien - insbesondere komplexen Hydriden - wie NaBH₄, Ca(BH₄)₂, LiAlH₄ und anderen Aluminium- oder Bor-Hydriden bei Temperaturen von 0 - 100°C und Drücken von 760 Torr aufwärts (Beispiel 1).

Die Verbindungen können darüberhinaus hergestellt werden aus Chlormethylverbindungen der Formel (II) oder entsprechenden Verbindungen mit nucleofuger Abgangsgruppe wie Halogen, Alkyl- oder Arylsulfonat, mit Aminoalkylphenolen (III) mit basischen Hilfsmitteln wie Hydroxiden, Alkoholaten, Carbonaten in polaren Lösungsmitteln wie DMF, Acetonitril oder Ethanol (Beispiel 2). 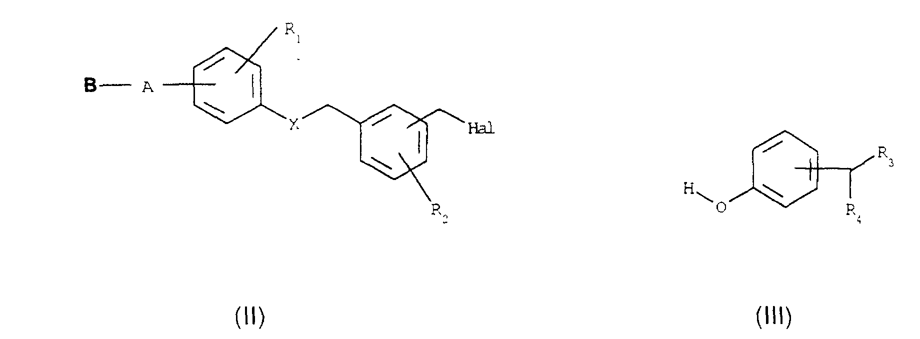 (R1 bis R4, sowie A, B und X wie eingangs definiert; Hal bedeutet in erster Linie Halogen oder einen Alkyl- bzw. Arylsulfonatrest)

### Beispiel 1

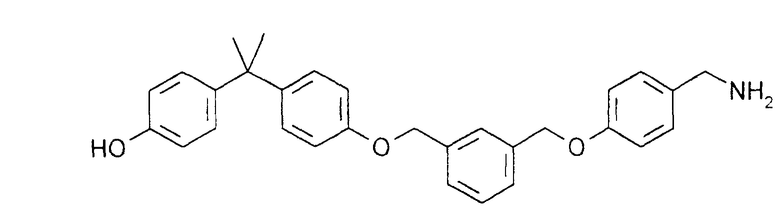

4-[[3-[[4-[1-(4-Hydroxyphenyl)-1-methylethyl]phenoxy]methyl]phenyl]methoxy]-benzyiamin hydrochlorid

2 g 4-[[3-[[4-[1-(4-Hydroxyphenyl)-1-methylethyl]phenoxy]methyl]phenyl]methoxy]-benzonitril wurden in 50 ml Methanol gelöst und Raney-Nickel hinzugegeben. Das Gemisch wurde 6 Std. bei Raumtemp. und Normaldruck hydriert. Der Katalysator wurde abgesaugt und das Lösungsmittel abdestilliert. Der Rückstand wurde in Methanol aufgenommen, mit ethanolischer Salzsäure angesäuert und das Produkt über Kieselgel mit Dichlormethan/Methanol 1:1 chromatographiert. Nach Kristallisation mit Essigester/Ether erhielt man 0.5 g Produkt als Hydrochlorid mit dem Schmelzpkt. 161-162°C.

### Beispiel 2

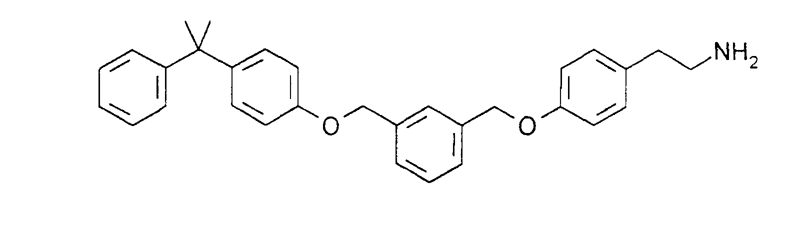

2-[4-[[3-[[4-[1-Phenyl-1-methylethyl]phenoxy]methyl]phenyl]methoxy]]-ethylamin hydrochlorid

1.15 g 4-Aminoethyl-phenol wurden in 15 ml Methanol gelöst und 1.5 g Natriummethylat als 30%ige Lösung in Methanol zugegeben. Das Solvens wurde abdestilliert und der Rückstand zu einer Lösung von 2.93 g 3-(4-(2-Phenylpropyl)-phenoxymethyl)-benzylchlorid in 25 ml Acetonitril gegeben. Die Mischung wurde 3 Std. bei 60-70°C gerührt. Das Lösungsmittel wurde abdestilliert, der Rückstand mit alkoholischer Salzsäure angesäuert und das Produkt mit Ether ausgefällt. Die Substanz wurde mit Dichlormethan/Methanol 7:3 chromatographiert. Ausbeute: 1g, Fp. 145°C.

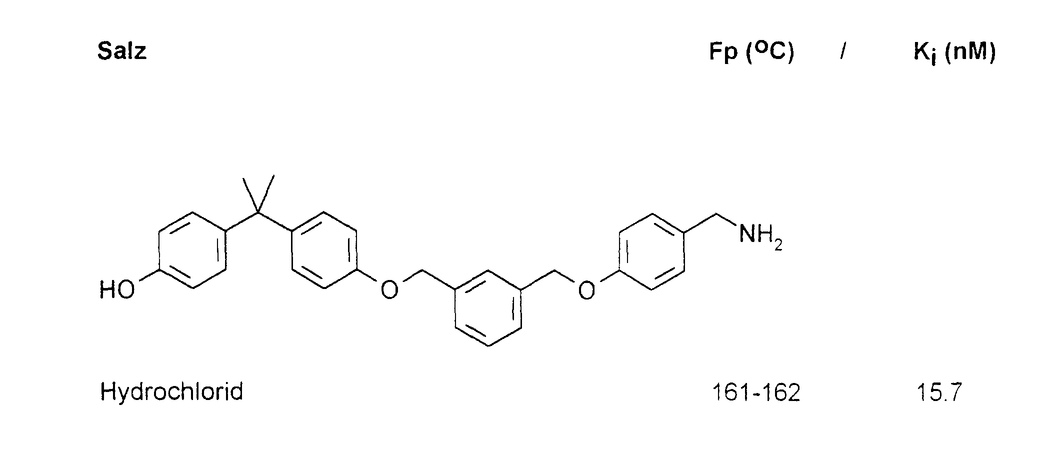

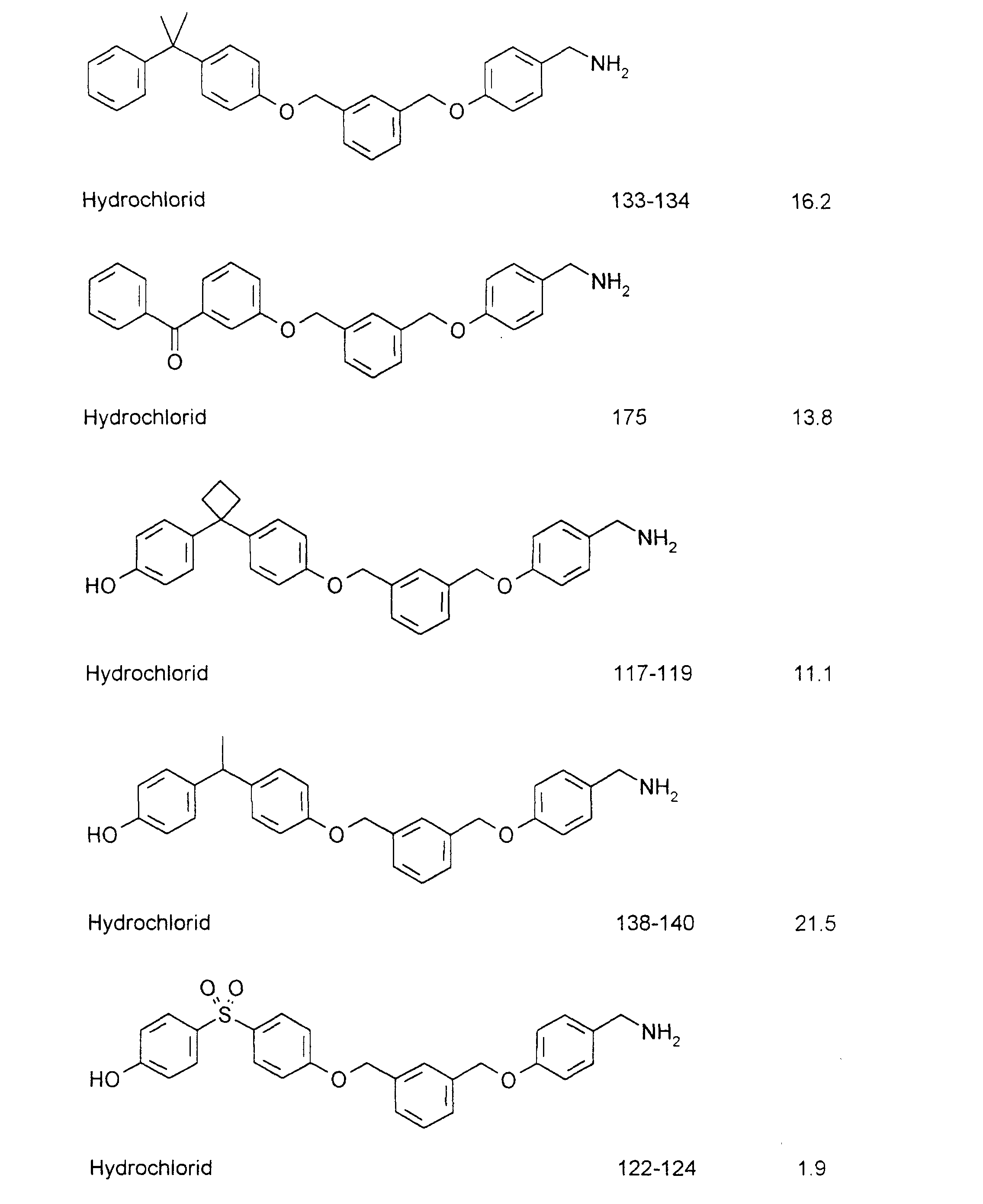

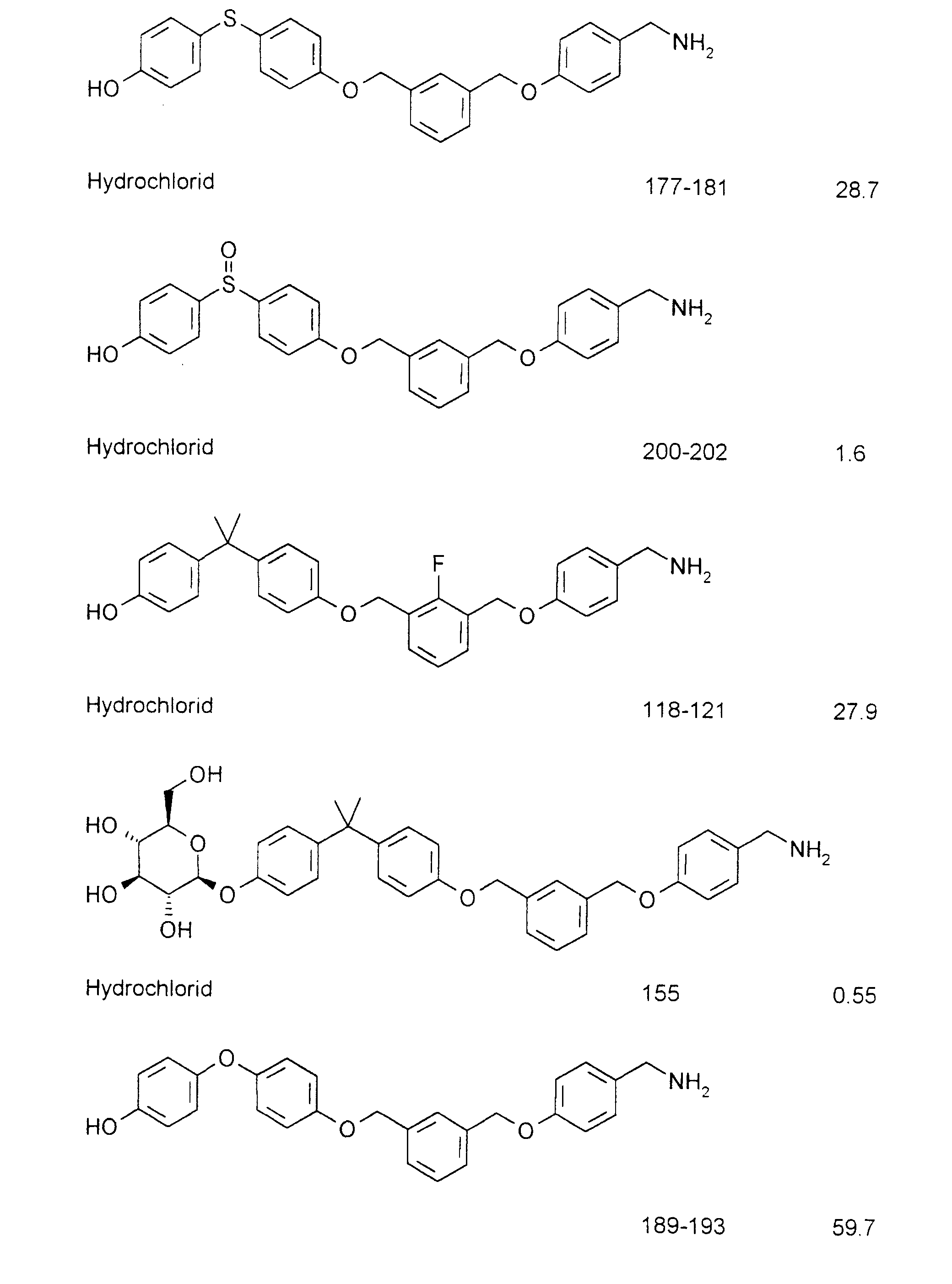

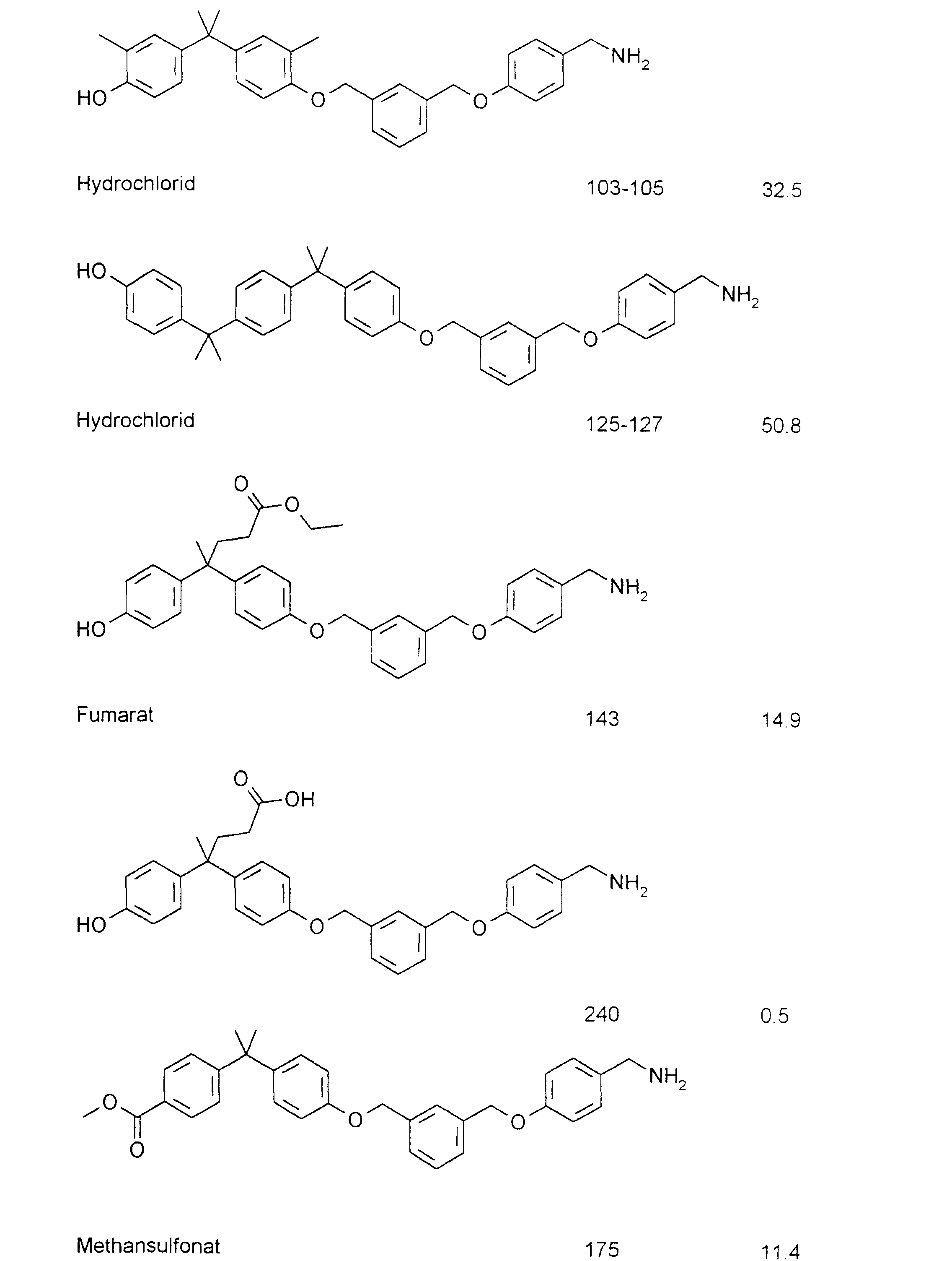

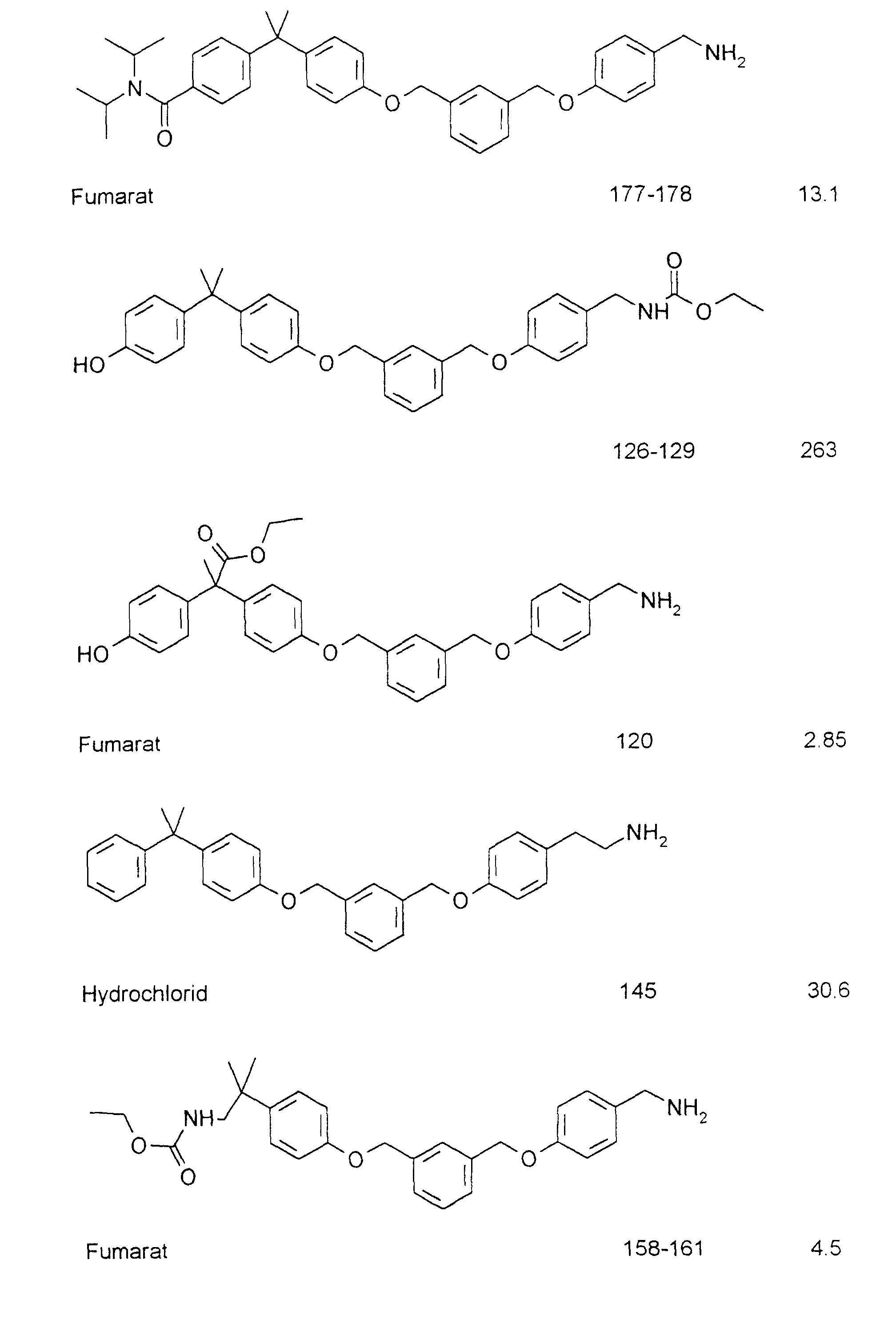

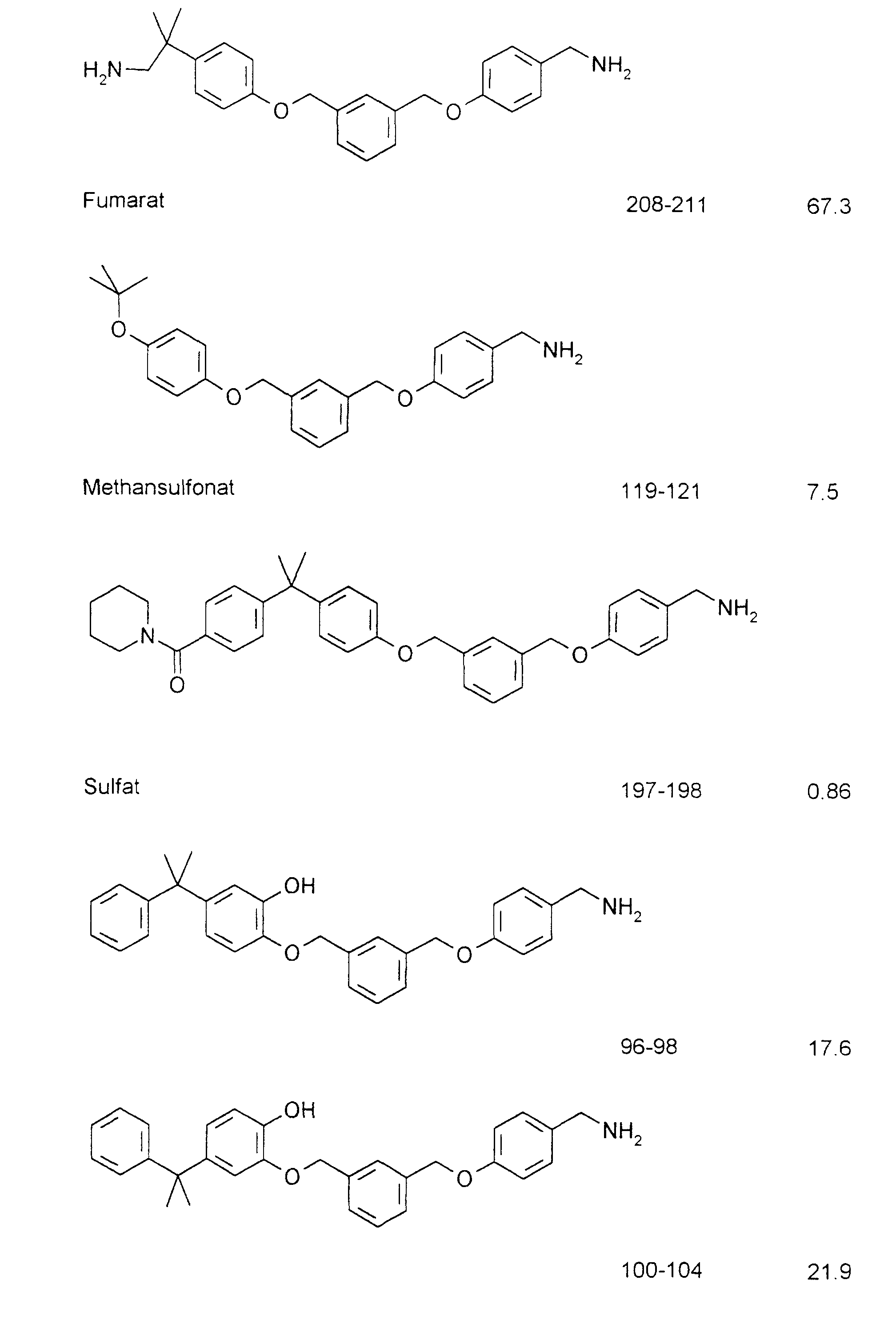

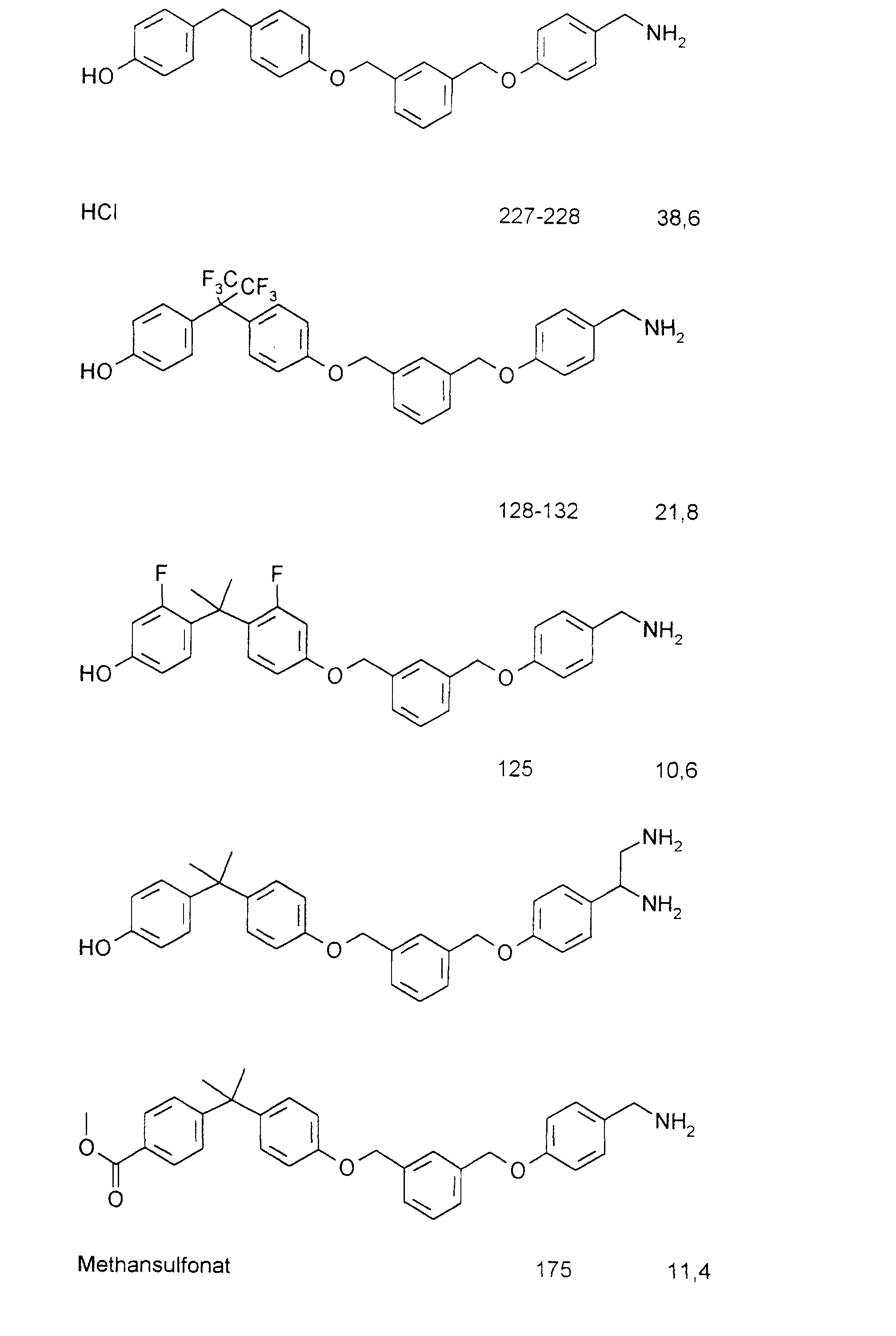

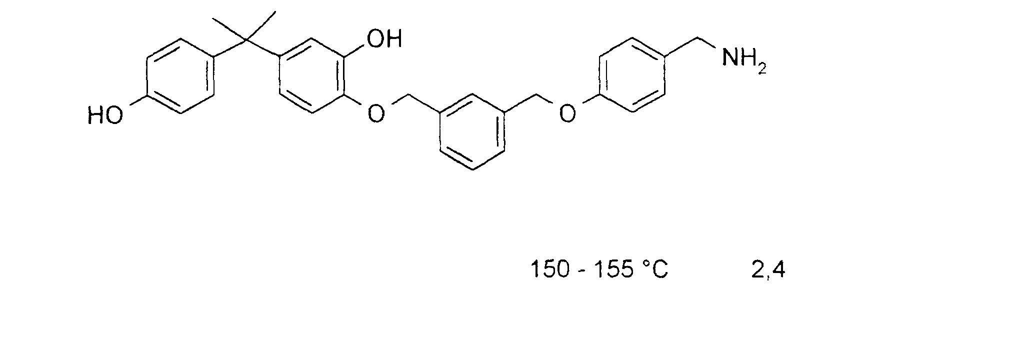

## Patentansprüche

1. Verbindungen der allgemeinen Formel I 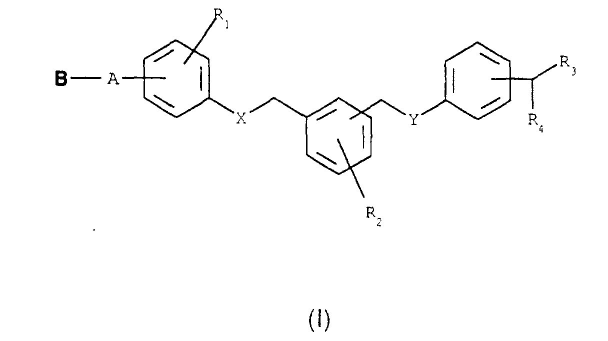 worin
X O, NH, N(CH₃), CH₂;
Y O, NH, N(CH₃), CH₂;
R₁ H, F, Cl, Br, J, C₁-C₆-Alkyl, OH, O-C₁-C₆-Alkyl, CF₃;
R₂ H, F, Cl, Br, J, C₁-C₆-Alkyl, OH, O-C₁-C₆-Alkyl, CF₃;
R₃ H, NH₂, NHCOR₅;
R₄ H, CH₂NH₂, CH₂NHCOR₅;
R₅ H, C₁-C₆-Alkyl, Phenyl, O-(C₁-C₆-Alkyl), wobei der Phenylring bis
zu zweimal substituiert sein kann durch: F, Cl, Br, J, Rₐ, ORₐ, CF₃,
Rₐ H, C₁-C₆-Alkyl;
A CR₆R₇, CO, SOₓ, O;
x eine ganze Zahl 0, 1 oder 2,
R₆ H, C₁-C₄-Alkyl, -(CH₂)_{y}COOR₈, CF₃, -(CH₂)_{y}OR₈, OR₈;
y eine ganze Zahl 0, 1, 2, 3 oder 4;
R₇ H, C₁-C₄-Alkyl, -(CH₂)_{z}COOR₈, -(CH₂)_{z}OR₈, CF₃;
z eine ganze Zahl 0, 1, 2, 3 oder 4,
wobei R₆ und R₇ miteinander ggf. einen C₃-C₆-Cycloalkylring bilden können;
R₈ H, C₁-C₆-Alkyl;
B C₁-C₆-Alkyl, Ar, CONR₉R₁₀ und, wenn A die Bedeutung -C(CH₃)₂ hat: CH₂NR₉R₁₀, CH₂NR₉COR₁₁;
Ar Phenyl, Naphthyl, Thienyl, Pyridyl - gegebenfalls bis zu zweimal substituiert mit R₁₂,
R₉ H, C₁-C₆-Alkyl;
R₁₀ H, C₁-C₆-Alkyl, wobei R₉ und R₁₀ zusammen mit dem Stickstoffatom einen Ring mit 3 bis 7 Kohlenstoffatomen bilden können;
R₁₁ H, C₁-C₆-Alkyl, -O-(C₁-C₆-Alkyl), Phenyl;
R₁₂ H, C₁-C₆-Alkyl, O-(C₁-C₆-Alkyl), F, Cl, Br, J, Rₐ, CF₃, CHF₂, C(CH₃)₂-phenylen-OH, COORₐ, CONRₐR_{b,} OR_{c};
Rₐ H, C₁-C₆-Alkyl;
R_{b} H, C₁-C₆-Alkyl, wobei ggf. Rₐ und R_{b} zusammen mit dem Stickstoffatom einen Ring mit 3 bis 7 Kohlenstoffatomen bilden können;
R_{c} für H, C₁-C₆-Alkyl, COOR_{d}, COR_{d} oder eine Gruppe der Formel 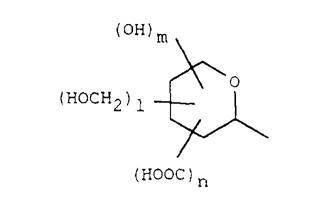 l, m, n eine ganze Zahl 0, 1, 2, 3 oder 4 wobei gilt l+m+n≤4;
R_{d} C₁-C₆-Alkyl, Phenyl
bedeuten können - gegebenenfalls in Form der einzelnen optischen Isomeren, Mischungen der einzelnen Enantiomeren oder Racemate sowie in Form der freien Basen oder der enstsprechenden Säureadditionssalze mit pharmakologisch unbedenklichen Säuren - mit der Maßgabe daß
R₃ und R₄ beide zusammen nicht Wasserstoff bedeuten können.

2. Verbindungen der allgemeinen Formel l nach Anspruch 1, in der
X O;
Y O;
R₁ H, F, Cl, Br, J, C₁-C₆-Alkyl, OH, O-C₁-C₆-Alkyl, CF₃;
R₂ H, F, Cl, Br, J, C₁-C₆-Alkyl, OH, O-C₁-C₆-Alkyl, CF₃;
R₃ NH₂;
R₄ H ;
A CR₆R₇, O;
R₆ H, C₁-C₄-Alkyl, CF₃;
R₇ H, C₁-C₄-Alkyl, CF₃,
wobei R₆ und R₇ miteinander ggf. einen C₃-C₆-Cycloalkylring bilden können;
B Phenyl, ggf. bis zu zweimal substituiert mit F, Cl, Br, J, Rₐ, OR_{c}, CF₃;
Rₐ H, C₁-C₆-Alkyl;
R_{c} für H, C₁-C₆-Alkyl, COOR_{d}, COR_{d} oder eine Gruppe der Formel 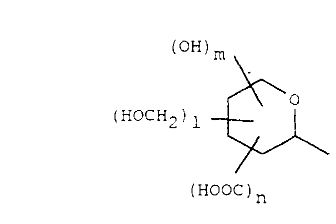 I, m, n eine ganze Zahl 0, 1, 2, 3 oder 4 wobei gilt I+m+n ≤ 4;
R_{d} C₁-C₆-Alkyl, Phenyl
bedeuten können - gegebenenfalls in Form der einzelnen optischen Isomeren, Mischungen der einzelnen Enantiomeren oder Racemate sowie in Form der freien Basen oder der entsprechenden Säureadditionssalze mit pharmakologisch unbedenklichen Säuren.

3. Verfahren zur Herstellung von Verbindungen der algemeinen Formel 1, **dadurch gekennzeichnet, daß** man eine Verbindung der Formel (II) 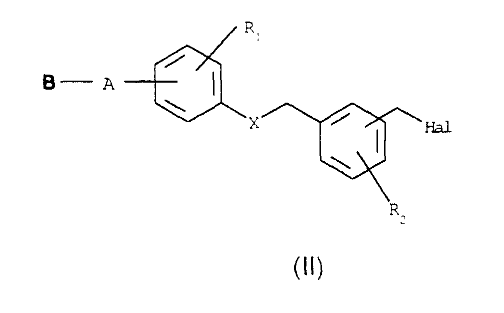 in der R₁ , R₂, A, B und X die in Anspruch 1 angegebene Bedeutung haben und Hal neben Halogen eine nucleofuge Abgangsgruppe, bevorzugt Chlor, Alkyl- oder Arylsulfonat bedeuten kann, mit einem Phenol oder Thiophenol der allgemeinen Formel III 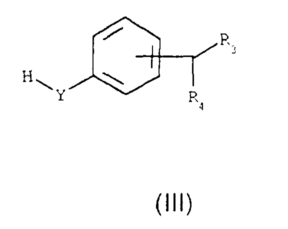 in der R₃, R₄ und Y wie in Anspruch 1 definiert sind, in Gegenwart einer basisch reagierenden Verbindung - bevorzugt in Gegenwart basisch reagierender Hydroxide, Alkoholate oder Carbonate von Alkali- oder Erdalkalimetallen - in einem polaren Lösungsmittel, bevorzugt Dimethylformamid, Acetonitril oder Ethanol, oder in einer Mischung dieser Lösungsmittel umsetzt. und das Reaktionsprodukt isoliert, gegebenfalls eine Racemtatrennung durchführt und - gewünschtenfalls - ein Säureadditionssalz mit einer pharmakologisch unbedenklichen Säure bildet.

4. Verfahren zur Herstellung von Verbindungen der allgemeinen Formel I, in der R₁, R₂, R₃ und R₄, sowie A, B, X und Y wie in Anspruch 1 definiert sind, **dadurch gekennzeichnet, daß** man auf an sich bekannte Weise zunächst ein entsprechendes Benzonitrilderivat der allgemeinen Formel IV 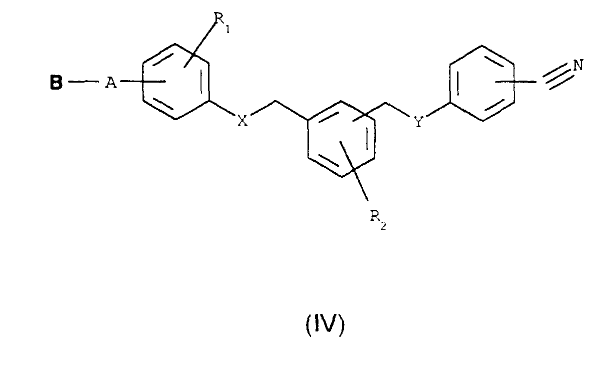 in einem Solvens aus der Gruppe Methanol, Ethanol oder in einem höheren Alkohol, DMF oder Wasser in Gegenwart eines Katalysators aus der Gruppe Raney-Nickel, Pd/C, Platin oder mit Hydrid-Reagenzien - insbesondere komplexen Hydriden aus der Gruppe NaBH₄, Ca(BH₄)₂, LiAlH₄ oder anderen Aluminium- oder Bor-Hydriden - bei Temperaturen in einem Intervall von 0 bis 100°C und unter einem Wasserstoffdruck von größer 760 Torr zum entsprechenden Amin der allgemeinen Formel I hydriert.

5. Pharmazeutische Zubereitung, **gekennzeichnet durch** einen Gehalt an einer Verbindung nach einem der Ansprüche 1 oder 2 und deren Säureadditionssalze neben üblichen Hilfs- und Trägerstoffen.

6. Verbindungen nach einem der Ansprüche 1 oder 2 als Arzneimittel.

7. Verwendung von Verbindungen nach einem der Ansprüche 1 oder 2 zur Herstellung eines Arzneimittels mit LTB₄-antagonistischer Wirkung

8. Verwendung von Verbindungen der allgemeinen Formel l, deren Stereoisomeren sowie deren Säureadditionssalzen zur Herstellung eines Medikaments zur therapeutischen Behandlung von Arthritis, Asthma, chronischer obstruktiver Lungenerkrankung wie chronischer Bronchitis, Psoriasis, Colitis ulcerosa, durch nichtsteroidale Antiphlogistika induzierter Gastro- oder Enteropathie, cystischer Fibrose, Alzheimer-Krankheit, Schock, Reperfusionsschäden/lschämien, Atherosklerose, multipler Sklerose.

## Claims

1. Compounds of general formula I 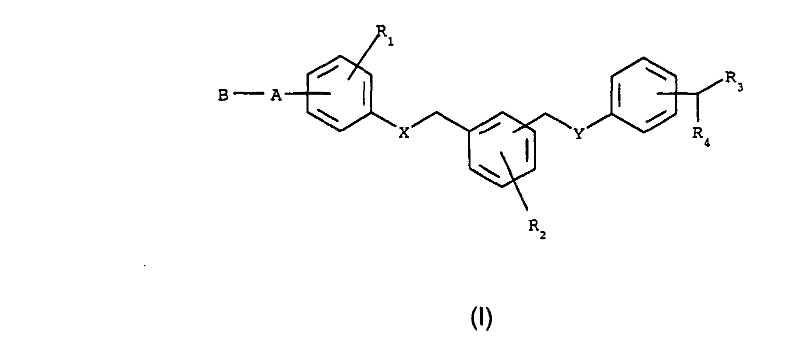 wherein
X denotes O, NH, N(CH₃), CH₂;
Y denotes O, NH, N(CH₃), CH₂;
R₁ denotes H, F, Cl, Br, I, C₁₋₆-alkyl, OH, O-C₁₋₆-alkyl, CF₃;
R₂ denotes H, F, Cl, Br, l, C₁₋₆-alkyl, OH, O-C₁₋₆-alkyl, CF₃;
R₃ denotes H, NH₂, NHCOR₅;
R₄ denotes H, CH₂NH₂, CH₂NHCOR₅;
R₅ denotes H, C₁₋₆-alkyl, phenyl, O-(C₁₋₆-alkyl), whilst the phenyl ring may be substituted up to twice by: F, Cl, Br, I, Rₐ, ORₐ, CF₃,
Rₐ denotes H, C₁₋₆-alkyl;
A denotes CR₆R₇, CO, SOₓ, O;
x denotes an integer 0, 1 or 2;
R₆ denotes H, C₁ -₄-alkyl, -(CH₂)_{y}COOR₈, CF₃, -(CH₂)_{y}OR₈, OR₈;
y denotes an integer 0, 1 , 2, 3 or 4;
R₇ denotes H, C₁₋₄-alkyl, -(CH₂)_{z}COOR₈, -(CH₂)_{z}OR₈, CF₃;
z denotes an integer 0, 1, 2, 3 or 4, whilst R₆ and R₇ together may optionally form a C₃₋₆-cycloalkyl ring;
R₈ denotes H, C₁₋₆-alkyl;
B denotes C₁₋₆-alkyl, Ar, CONR₉R₁₀, and, if A represents -C(CH₃)₂: CH₂NR₉R₁₀, CH₂NR₉COR₁₁;
Ar denotes phenyl, naphthyl, thienyl, pyridyl - optionally twice substituted up to with R₁₂,
R₉ denotes H, C₁₋₆-alkyl;
R₁₀ denotes H, C₁₋₆-alkyl, whilst R₉ and R₁₀ together with the atom nitrogen may form a ring having 3 to 7 carbon atoms;
R₁₁ denotes H, C₁₋₆-alkyl, -O-(C₁₋₆-alkyl), phenyl;
R₁₂ denotes H, C₁₋₆-alkyl, O-(C₁₋₆-alkyl), F, Cl, Br, I, Rₐ, CF₃, CHF₂, C(CH₃)₂-phenylene-OH, COORₐ, CONRₐR_{b}, OR_{c};
Rₐ denotes H, C₁₋₆-alkyl;
R_{b} denotes H, C₁₋₆-alkyl, whilst optionally Rₐ and R_{b} together with the nitrogen atom may form a ring having 3 to 7 carbon atoms;
R_{c} denotes H, C₁₋₆-alkyl, COOR_{d}, COR_{d} or a group of formula 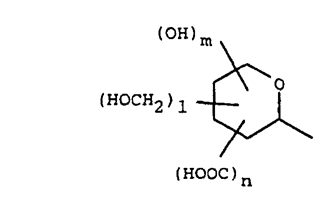 l, m, n denote an integer 0, 1, 2, 3 or 4 whilst l+m+n ≤ 4;
R_{d} denotes C₁₋₆-alkyl, phenyl,
optionally in the form of the individual optical isomers, mixtures of the individual enantiomers or racemates and in the form of the free bases or the corresponding acid addition salts with pharmacologically acceptable acids - with the proviso that R₃ and R₄ together cannot denote hydrogen.

2. Compounds of general formula I according to claim 1 wherein
X denotes O;
Y denotes O;
R₁ denotes H, F, Cl, Br, I, C₁₋₆-alkyl, OH, O-C₁₋₆-alkyl, CF₃;
R₂ denotes H, F, Cl, Br, l, C₁₋₆₋alkyl, OH, O-C₁₋₆-alkyl, CF₃;
R₃ denotes NH₂;
R₄ denotes H;
A denotes CR₆R₇, O;
R₆ denotes H, C₁₋₄-alkyl, CF₃;
R₇ denotes H, C₁₋₄-alkyl, CF₃,
whilst R₆ and R₇ together may optionally form a C₃-C₆-cycloalkyl ring;
B denotes phenyl, optionally substituted up to twice with F, Cl, Br, I, Rₐ, OR_{c}, CF₃;
Rₐ denotes H, C₁-₆-alkyl;
R_{c} denotes H, C₁-₆-alkyl, COOR_{d}, COR_{d} or a group of formula 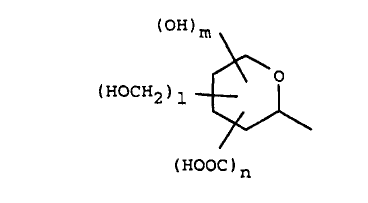 I, m, n denote an integer 0, 1, 2, 3 or 4 whilst I+m+n≤ 4;
R_{d} denotes C₁₋₆-alkyl, phenyl
optionally in the form of the individual optical isomers, mixtures of the individual enantiomers or racemates and in the form of the free bases or the corresponding acid addition salts with pharmacologically acceptable acids.

3. Process for preparing compounds of general formula I, **characterised in that** a compound of formula (II) 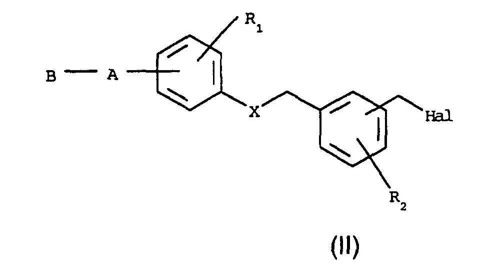 wherein R₁, R₂, A, B and X are defined as in claim 1
and Hal may represent, in addition to halogen, a nucleofugic leaving group, preferably chlorine, alkyl sulphonate or aryl sulphonate, is reacted with a phenol or thiophenol of general formula III 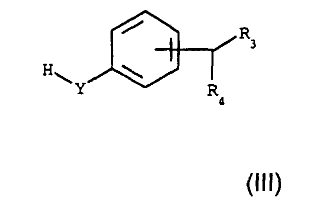 wherein R₃, R₄ and Y are defined as in claim 1, in the presence of a basic reacting compound, preferably in the presence of basic reacting hydroxides, alkoxides or carbonates of alkali or alkaline earth metals, in a polar solvent, preferably dimethylformamide, acetonitrile or ethanol, or in a mixture of these solvents, and the reaction product is isolated, racemate separation is optionally carried out, and - if desired - an acid addition salt is formed with a pharmacologically acceptable acid.

4. Process for preparing compounds of general formula l wherein R₁, R₂, R₃ and R₄ and also A, B, X and Y are defined as in claim 1,
**characterised in that** first of all a corresponding benzonitrile derivative of general formula IV 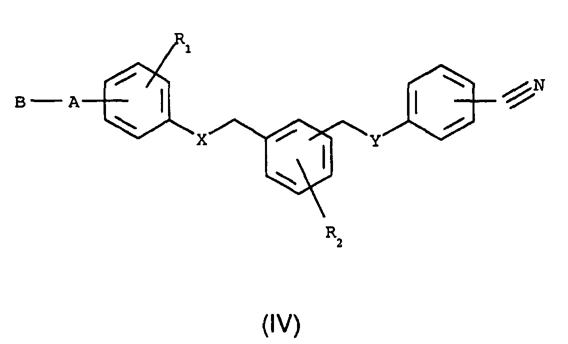 is hydrogenated in a manner known *per se,* in a solvent chosen from the group methanol, ethanol or a higher alcohol, DMF or water in the presence of a catalyst chosen from the group Raney nickel, PD/C, platinum, or with hydride reagents, particularly complex hydrides chosen from the group NaBH₄, Ca(BH₄)₂, LiAlH₄ or other aluminium or boron hydrides, at temperatures in the range from 0 to 100°C and at a hydrogen pressure of greater than 760 Torr, to form the corresponding amine of general formula I.

5. Pharmaceutical preparation, **characterised in that** it contains a compound according to one of claims 1 and 2 and the acid addition salts thereof together with conventional excipients and carriers.

6. Compounds according to one of claims 1 and 2 as pharmaceutical compositions.

7. Use of compounds according to one of claims 1 and 2 for preparing a pharmaceutical preparation with an LTB₄-antagonistic activity.

8. Use of compounds of general formula I, their stereoisomers and acid addition salts for preparing a medicament for the therapeutic treatment of arthritis, asthma, chronic obstructive lung disease such as chronic bronchitis, psoriasis, ulcerative colitis, gastropathy or enteropathy induced by nonsteroidal antiinflammatories, cystic fibrosis, Alzheimer's disease, shock, reperfusion damage/ischaemias, atherosclerosis and multiple sclerosis.

## Revendications

1. Composés de formule générale I 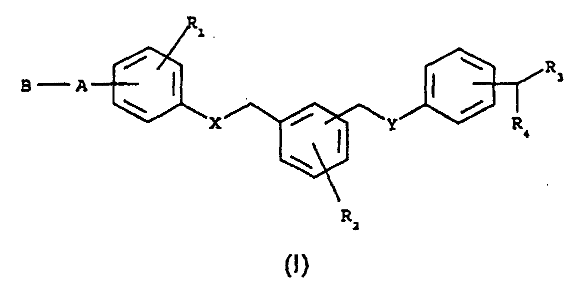 où
X peut représenter O, NH, N(CH₃), CH₂ ;
Y peut représenter O, NH, N (CH₃) , CH₂ ;
R₁ peut représenter H, F, Cl, Br, I, alkyle en C₁-C₆, OH, O-alkyle en C₁-C₆, CF₃ ;
R₂ peut représenter H, F, Cl, Br, I, alkyle en C₁-C₆, OH, O-alkyle en C₁-C₆, CF₃ ;
R₃ peut représenter H, NH₂, NHCOR₅ ;
R₄ peut représenter H, CH₂NH₂, CH₂NHCOR₅ ;
R₅ peut représenter H, alkyle en C₁-C₆, phényle, O-alkyle en C₁-C₆, où le cycle phényle peut être substitué jusqu'à deux fois par : F, Cl, Br, I, Rₐ, ORₐ, CF₃ ;
Rₐ peut représenter H, alkyle en C₁-C₆ ;
A peut représenter CR₆R₇, CO, SOₓ, O ;
x peut représenter un nombre entier 0, 1 ou 2 ;
R₆ peut représenter H, alkyle en C₁-C₄, - (CH₂) _{y}COOR₈, CF₃ , ―( CH₂) _{y}OR₈ , OR₈ ;
y peut représenter un nombre entier 0, 1, 2, 3 ou 4 ;
R₇ peut représenter H, alkyle en C₁-C₄, -(CH₂)_{z}COOR₈, -(CH₂)_{z}OR₈, CF₃ ;
z peut représenter un nombre entier 0, 1, 2, 3 ou 4 ; où R₆ et R₇ peuvent éventuellement former ensemble un cycle cycloalkyle en C₃-C₆;
R₈ peut représenter H, alkyle en C₁-C₆ ;
B peut représenter alkyle en C₁-C₆, Ar, CONR₉R₁₀ et, quand A a la signification -C (CH₃)₂ : CH₂NR₉R₁₀, CH₂NR₉COR₁₁ ;
Ar peut représenter phényle, naphtyle, thiényle, pyridyle, éventuellement substitué jusqu'à deux fois par R₁₂,
R₉ peut représenter H, alkyle en C₁-C₆ ;
R₁₀ peut représenter H, alkyle en C₁-C₆, où R₉ et R₁₀ peuvent former avec l'atome d'azote un cycle à 3 à 7 atomes de carbone ;
R₁₁ peut représenter H, alkyle en C₁-C₆, -O-alkyle en C₁-C₆, phényle ;
R₁₂ peut représenter H, alkyle en C₁-C₆, -O-alkyle en C₁-C₆, F, Cl, Br, I, Rₐ, CF₃, CHF₂, C(CH₃)₂-phénylène-OH, COORₐ, CONRₐR_{b}, ORc ;
Rₐ peut représenter H, alkyle en C₁-C₆;
R_{b} peut représenter H, alkyle en C₁-C₆, où éventuellement Rₐ et R_{b} peuvent former avec l'atome d'azote un cycle à 3 à 7 atomes de carbone ;
R_{c} peut représenter H, alkyle en C₁-C₆, COOR_{d}, COR_{d} ou un groupe de formule 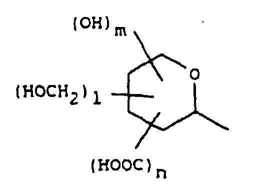 l, m, n peuvent représenter un nombre entier 0, 1, 2, 3 ou 4 où l+m+n≤4 ;
R_{d} peut représenter alkyle en C₁-C₆, phényle,
éventuellement sous forme des différents isomères optiques, de mélanges des différents énantiomères ou racémates et sous forme des bases libres ou des sels d'addition d'acide correspondants avec des acides pharmacologiquement irréprochables, avec la condition que R₃ et R₄ ne peuvent pas représenter ensemble l'hydrogène.

2. Composés de formule générale I selon la revendication 1 où
X peut représenter O ;
Y peut représenter O ;
R₁ peut représenter H, F, Cl, Br, I, alkyle en C₁-C₆, OH, O-alkyle en C₁-C₆, CF₃ ;
R₂ peut représenter H, F, Cl, Br, I, alkyle en C₁-C₆, OH, O-alkyle en C₁-C₆, CF₃ ;
R₃ peut représenter NH₂ ;
R₄ peut représenter H ;
A peut représenter CR₆R₇, O ;
R₆ peut représenter H, alkyle en C₁-C₄, CF₃ ;
R₇ peut représenter H, alkyle en C₁-C₄, CF₃ ;
où R₆ et R₇ peuvent éventuellement former ensemble un cycle cycloalkyle en C₃-C₆ ;
B peut représenter phényle, éventuellement substitué jusqu'à deux fois par F, Cl, Br, I, Rₐ, OR_{c}, CF₃ ;
Rₐ peut représenter H, alkyle en C₁-C₆;
R_{c} peut représenter H, alkyle en C₁-C₆, COOR_{d}, COR_{d} ou un groupe de formule 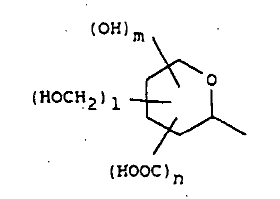 l, m, n peuvent représenter un nombre entier 0, 1, 2, 3 ou 4 où l+m+n<4 ;
R_{d} peut représenter alkyle en C₁-C₆, phényle,
éventuellement sous forme des différents isomères optiques, de mélanges des différents énantiomères ou racémates et sous forme des bases libres ou des sels d'addition d'acide correspondants avec des acides pharmacologiquement irréprochables.

3. Procédé de préparation de composés de formule générale I **caractérisé en ce que** l'on fait réagir un composé de formule (II) 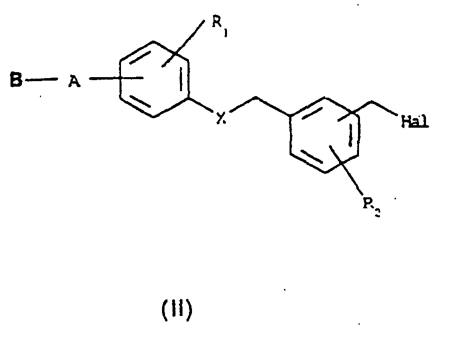 où R₁, R₂, A, B et X ont la signification indiquée dans la revendication 1 et Hal peut représenter, outre un halogène, un groupe partant nucléofuge, de préférence le chlore, alkyl- ou arylsulfonate, avec un phénol ou thiophénol de formule générale III 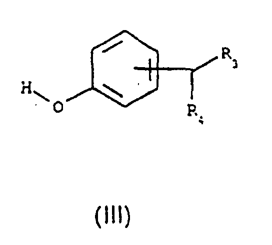 où R₃, R₄ et Y sont définis comme dans la revendication 1, en présence d'un composé à réaction basique, de préférence en présence d'hydroxydes, alcoolates ou carbonates de métaux alcalins ou alcalinoterreux à réaction basique, dans un solvant polaire, de préférence le diméthylformamide, l'acétonitrile ou l'éthanol, ou dans un mélange de ces solvants, et on isole le produit réactionnel, on réalise éventuellement une résolution de racémate et, si on le souhaite, on forme un sel d'addition d'acide avec un acide pharmacologiquement irréprochable.

4. Procédé de préparation de composés de formule générale I où R₁, R₂, R₃ et R₄, ainsi que A, B, X et Y, sont définis comme dans la revendication 1, **caractérisé en ce que** l'on hydrogène d'abord de manière connue en soi un dérivé de benzonitrile correspondant de formule générale IV 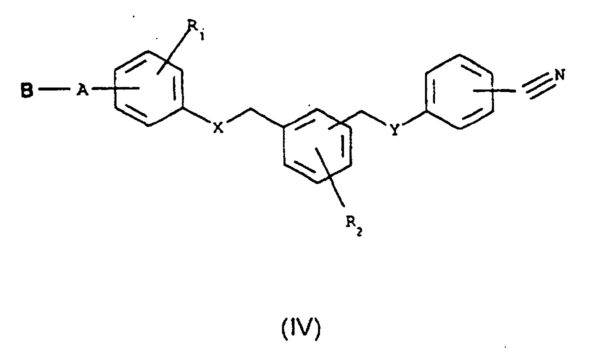 dans un solvant du groupe méthanol, éthanol ou dans un alcool supérieur, le DMF ou l'eau en présence d'un catalyseur du groupe nickel de Raney, Pd/C, platine ou avec des réactifs hydrures, en particulier des hydrures complexes du groupe NaBH₄, Ca(BH₄)₂, LiAlH₄ ou d'autres hydrures d'aluminium ou de bore, à des températures dans un intervalle de 0 à 100°C et sous une pression d'hydrogène supérieure à 760 torr en l'amine de formule générale I correspondante.

5. Préparation pharmaceutique **caractérisée par** une teneur en un composé selon l'une des revendications 1 ou 2 et ses sels d'addition d'acide, outre des adjuvants et supports courants.

6. Composés selon l'une des revendications 1 ou 2 comme médicament.

7. Utilisation de composés selon l'une des revendications 1 ou 2 pour la production d'un médicament à effet antagoniste de LTB₄.

8. Utilisation de composés de formule générale I, de leurs stéréoisomères et de leurs sels d'addition d'acide pour la production d'un médicament pour le traitement thérapeutique de l'arthrite, de l'asthme, des maladies pulmonaires obstructives chroniques comme la bronchite chronique, du psoriasis, de la colite ulcéreuse, de la gastro- ou entéropathie induite par des antiphlogistiques non stéroïdiens, de la fibrose kystique, de la maladie d'Alzheimer, du choc, des lésions de reperfusion/ischémies, de l'athérosclérose, de la sclérose en plaques.
